# EUROPEAN PATENT APPLICATION

(11) **EP 4 516 313 A1**
(43) Date of publication of application: **05.03.2025**
(21) Application number: 23796458.0
(22) Date of filing: 27.04.2023
(51) Int. Cl.: A61K 39/39, A61K 31/715, A61K 36/06, A61K 36/484, A61P 31/00, A61P 35/00, A61P 37/04

(54) **IMMUNOACTIVATOR, VACCINE ADJUVANT, AND METHOD FOR INDUCING IMMUNITY**

(30) Priority: 28.04.2022 JP 2022074927
(71) Applicant: Teikyo University, Tokyo 173-8605 (JP)
(72) Inventor: SUZUKI Ryo, Tokyo 173-8605 (JP); OMATA Daiki, Tokyo 173-8605 (JP); MUNAKATA Lisa, Tokyo 173-8605 (JP); SUZUKI Yuno, Tokyo 173-8605 (JP); KOIZUMI Keiichi, Toyama-shi, Toyama 930-0194 (JP)
(74) Representative: Hoffmann Eitle
(86) International application number: PCT/JP2023/016572
(87) International publication number: WO 2023/210722

(57) **Abstract**

Dislcosed is an immunoactivator that is capable of enhancing induction of both humoral immunity and cellular immunity and contains an active ingredient derived from cell walls (derived from a natural product); a vaccine adjuvant; and a method for inducing immunity including administering the immunoactivator. Theimmunoactivator contains, as an active ingredient, particles having a maximum diameter within a range of 1 to 800 nm, wherein the particles comprise cell-wall-derived polysaccharides.

## Description

### TECHNICAL FIELD

The present disclosure relates to: an immunoactivator containing, as an active ingredient, particles that comprise cell-wall-derived polysaccharides and have a maximum diameter of 1 to 800 nm; a vaccine adjuvant containing the above immunoactivator; and a method for inducing immunity including a step of administering the above immunoactivator.

### BACKGROUND ART

In medical fields such as infection treatment and cancer immunotherapy, various preventive measures and therapeutic methods have been investigated so far for enhancing immune activity. For example, PTL 1 proposes to use an immunoactivator having a high cellular immunity induction effect. In these medical fields, various adjuvants to be used in the preventive measures or therapeutic methods have also been developed so far. Under such circumstances, further development of an immunoactivator having both efficacy of activating immune function and safety for human bodies is desired.

### RELATED ART DOCUMENT

### PATENT DOCUMENT

PTL 1: JP-A-2020-090445

### SUMMARY

### PROBLEMS TO BE SOLVED BY THE DISCLOSURE

Under such background, the present disclosure provides an immunoactivator capable of enhancing induction of both humoral immunity and cellular immunity, wherein, additionally, the active ingredient of the immunoactivator is derived from cell walls (derived from a natural product), so that consumers can feel a sense of safety; a vaccine adjuvant containing the above immunoactivator; and a method for inducing immunity including a step of administering the above immunoactivator.

### MEANS FOR SOLVING THE PROBLEMS

In view of the above situation, at first, the present inventors searched for substances that are obtained from natural products. In particular, they focused on the fact that recently found particles having a small particle diameter that are obtained from a natural product have various properties; and they made further research. As a result, among natural products, they found that particles that comprise cell-wall-derived polysaccharides and have a maximum diameter within a range of 1 to 800 nm can enhance induction of humoral immunity and cellular immunity.

That is, the present disclosure includes the following aspects.
[i] An immunoactivator comprising particles having a maximum diameter within a range of 1 to 800 nm as an active ingredient, wherein the particles comprise cell-wall-derived polysaccharides.
[ii] The immunoactivator as recited in [i], wherein the particles are derived from at least one selected from the group consisting of licorice, yeast, and lactic acid bacteria.
[iii] The immunoactivator as recited in [i] or [ii], wherein the immunoactivator is capable of at least one of infection treatment and cancer immunotherapy.
[iv] The immunoactivator as recited in any one of [i] to [iii], wherein the immunoactivator induces at least one of humoral immunity and cellular immunity.
[v] The immunoactivator as recited in any one of [i] to [iv], wherein the immunoactivator is capable of being administered by at least one administration method selected from the group consisting of subcutaneous administration, intradermal administration, intramuscular administration, pulmonary administration, intravascular administration, nasal administration, and oral administration.
[vi] A vaccine adjuvant comprising particles having a maximum diameter within a range of 1 to 800 nm as an active ingredient, wherein the particles comprise cell-wall-derived polysaccharides.
[vii] The vaccine adjuvant as recited in [vi], wherein the vaccine adjuvant is capable of being administered by at least one administration method selected from the group consisting of subcutaneous administration, intradermal administration, intramuscular administration, pulmonary administration, intravascular administration, nasal administration, and oral administration.
[viii] A method for inducing immunity including a step of administering the immunoactivator as recited in any one of [i] to [v] to a subject in need thereof.
[ix] The method for inducing immunity as recited in [viii], wherein the step of administering the immunoactivator includes administration of the immunoactivator by at least one administration method selected from the group consisting of subcutaneous administration, intradermal administration, intramuscular administration, pulmonary administration, intravascular administration, nasal administration, and oral administration.

### EFFECTS OF THE DISCLOSURE

The immunoactivator of the present disclosure highly induces both humoral immunity and cellular immunity. By a synergistic effect of both types of immunity, higher immune function activation can be exhibited. Besides, the active ingredient of the immunoactivator is derived from a natural product, so that users can feel a greater sense of safety.

Further, by using the vaccine adjuvant of the present disclosure with an antigen, the immune activity (immunogenicity) can be enhanced.

Furthermore, by the method for inducing immunity of the present disclosure, the effects of humoral immunity and cellular immunity can be obtained only via simple steps. Thus, the above method is safe and easy to use.

### BRIEF DESCRIPTION OF DRAWINGS

FIG. 1 is a transmission electron microscope photograph of a particle according to an embodiment of the present disclosure;
FIG. 2 is a graph showing particle size distribution of particles derived from licorice according to an embodiment of the present disclosure;
FIG. 3 is a graph showing particle size distribution of particles derived from yeast according to an embodiment of the present disclosure;
FIG. 4 is a graph showing particle size distribution of particles derived from lactic acid bacteria according to an embodiment of the present disclosure;
FIG. 5 is a graph showing results of cell experiments (maturation of dendritic cells) on the particles derived from licorice (CD40);
FIG. 6 is a graph showing results of cell experiments (maturation of dendritic cells) on the particles derived from licorice (CD80);
FIG. 7 is a graph showing results of cell experiments (maturation of dendritic cells) on the particles derived from licorice (CD86);
FIG. 8 shows a graph showing comparison of the results of FIG. 5, FIG. 6, and FIG. 7;
FIG. 9 is a graph showing analysis results of cell experiments (maturation of dendritic cells) on the particles derived from lactic acid bacteria (CD40);
FIG. 10 is a graph showing results of animal experiments (subcutaneous immunization: antigen-specific antibody production) on the particles derived from licorice (total IgG);
FIG. 11 is a graph showing results of animal experiments (subcutaneous immunization: antigen-specific antibody production) on the particles derived from licorice (IgG₁);
FIG. 12 is a graph showing results of animal experiments (subcutaneous immunization: antigen-specific antibody production) on the particles derived from licorice (IgG_{2c});
FIG. 13 is a graph showing results of animal experiments (subcutaneous immunization: antigen-specific antibody production) on the particles derived from licorice (Examples 8 and 9);
FIG. 14 is a graph showing results of animal experiments (subcutaneous immunization: antigen-specific antibody production) on the particles derived from yeast (OVA 1µg);
FIG. 15 is a graph showing results of animal experiments (subcutaneous immunization: antigen-specific antibody production) on the particles derived from yeast (OVA 10µg);
FIG. 16 is a graph showing results of animal experiments (subcutaneous immunization: T-cell response) on the particles derived from licorice;
FIG. 17 is a graph showing results of animal experiments (subcutaneous immunization: T-cell response) on the particles derived from yeast (OVA 1µg);
FIG. 18 is a graph showing results of animal experiments (subcutaneous immunization: T-cell response) on the particles derived from yeast (OVA 10µg);
FIG. 19 is a graph showing results of animal experiments (subcutaneous immunization: antitumor immunity induction 1) on the particles derived from licorice (Example 15);
FIG. 20 is a graph showing results of animal experiments (subcutaneous immunization: antitumor immunity induction 1) on the particles derived from licorice (Comparative Example 16);
FIG. 21 is a graph showing results of animal experiments (subcutaneous immunization: antitumor immunity induction 1) on the particles derived from licorice (Comparative Example 17);
FIG. 22 is a graph showing results of animal experiments (subcutaneous immunization: antitumor immunity induction 2) on the particles derived from licorice (E.G7-OVA cells);
FIG. 23 is a graph showing results of animal experiments (subcutaneous immunization: antitumor immunity induction 2) on the particles derived from licorice (MC38 cells);
FIG. 24 is a graph showing results of animal experiments (nasal immunization: antigen-specific antibody production) on the particles derived from licorice (total IgG);
FIG. 25 is a graph showing results of animal experiments (nasal immunization: antitumor immunity induction 1) on the particles derived from licorice (Example 17);
FIG. 26 is a graph showing results of animal experiments (nasal immunization: antitumor immunity induction 1) on the particles derived from licorice (Comparative Example 20);
FIG. 27 is a graph showing results of animal experiments (nasal immunization: antitumor immunity induction 1) on the particles derived from licorice (Comparative Example 21);
FIG. 28 is a graph showing results of animal experiments (nasal immunization: antitumor immunity induction 2) on the particles derived from licorice (E.G7-OVA cells); and
FIG. 29 is a graph showing results of animal experiments (nasal immunization: antitumor immunity induction 2) on the particles derived from licorice (MC38 cells).

### EMBODIMENTS OF THE DISCLOSURE

Hereinafter, the present disclosure will be specifically described.

However, the present disclosure is not limited to the embodiments described in the following.

In the present disclosure, the expression "X to Y" (X and Y are any numbers), unless otherwise specified, means "equal to or more than X and equal to or less than Y" and also encompasses the meaning of "preferably greater than X" or "preferably less than Y."

In addition, the expression "X and/or Y (X and Y are any configurations)" means at least one of X and Y, and includes the three meanings of "only X," "only Y," and "X and Y."

### <Immunoactivator>

The immunoactivator of the present disclosure contains, as an active ingredient, particles that comprise cell-wall-derived polysaccharides and have a maximum diameter within a range of 1 to 800 nm. Further, the immunoactivator of the present disclosure can be widely used in the medical field, health improvement field, and the like. In particular, it can be effectively used in infection treatment and cancer immunotherapy. Also, antiallergic drugs and immunomodulators are included in its concept.

In the present disclosure, the active ingredient means an ingredient capable of "activating immune function," which is an effect of the present disclosure. Provided that, even when an ingredient having the effect of "activating immune function" is contained, when the concentration is such that the effect is not produced, such component is excluded.

At first, the particles as an active ingredient of the present disclosure are described. The particles comprise cell-wall-derived polysaccharides, and the cell wall is found in cells of organisms belonging to the plant kingdom, organisms belonging to Fungi, organisms belonging to Protista, organisms belonging to Monera, and the like. Among the above organisms, preferred are original plants, which are the raw material of crude drugs; ascomycete; basidiomycete; and bacteria. Among them, Glycyrrhiza uralensis, Zingiber officinale, yeast, lactic acid bacteria, or the like are preferred.

Examples of the polysaccharides include cellulose, hemicellulose, and pectin. Examples of a constituent sugar of the polysaccharides include glucose, xylose, galactose, fucose, cellotriose, cellotetraose, xylan, arabinose, mannose, and rhamnose.

The particles as an active ingredient of the present disclosure have a maximum diameter of 1 to 800 nm, preferably 10 to 800 nm, more preferably 30 to 500 nm, and still more preferably 40 to 300 nm. The shape of the particles is, but not limited to, spherical, ellipsoidal, or disk-shaped (erythrocyte-like shape). Among these, spherical shape is preferred. In the present disclosure, "maximum particle diameter" refers to the particle diameter when the particles are spherical, and in the case of other shapes, it refers to the maximum length of the particles. The particle diameter can be measured as follows, for example: the obtained particles are dispersed in ultrapure water, and then the obtained dispersion liquid is measured by using dynamic light scattering. When the particle diameter is measured by using dynamic light scattering, the calculated average particle diameter is regarded as the particle maximum diameter. The calculated average particle diameter only needs to be within the range defined as the maximum diameter.

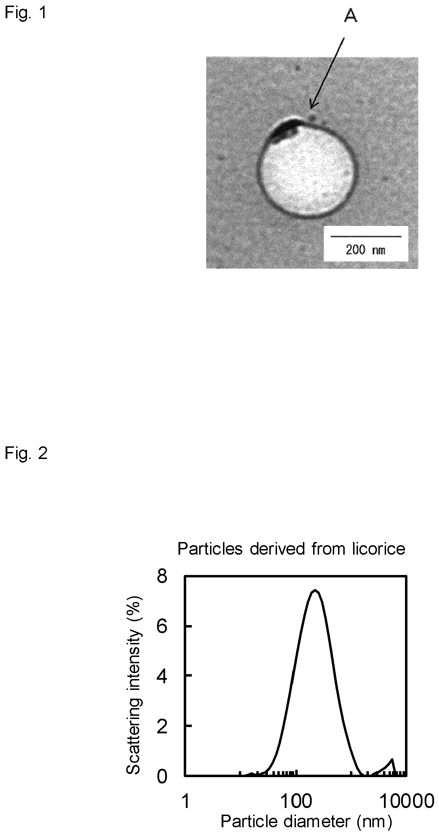

The particles typically have a shape as shown by symbol A in the electron microscope image of FIG. 1, and preferably have a spherical shape. The spherical shape includes not only a true spherical shape, but also an ellipsoidal shape. The particle A in FIG. 1 is derived from licorice. The negative-stained particle A is imaged by a transmission electron microscope.

When observed with an electron microscope, the structure of the particle A appears to have a bilayer structure, bilayer membrane structure, multi-layer structure, or multiple membrane structure. Accordingly, the particles as an active ingredient of the present disclosure are considered to have different electron density at least between the outermost layer and inner part.

The particles as an active ingredient of the present disclosure have solubility resistance to both aqueous and oily liquid, and have high dispersibility. In particular, the particles have high dispersibility in aqueous liquid. Furthermore, the excellent dispersibility is maintained for a long term. The dispersibility of the particles can be determined by, for example, dispersing the obtained particles in ultrapure water, capturing an image of the dispersion liquid with a transmission electron microscope, and then observing the degree of dispersion. Furthermore, the degree of dispersibility retention can be determined by comparing the dispersibility of the dispersion liquid before and after preservation for a certain period of time.

The particles as an active ingredient of the present disclosure have excellent barotolerance and heat resistance, and there is almost no change in the particle diameter by applying pressure up to at least 2 atm and heating up to 121°C. The barotolerance and heat resistance of particles can be determined as follows, for example: by calculating the particle diameter distribution of particles dispersed in ultrapure water and particles contained in the above dispersion liquid after being pressurized and heated, respectively, using dynamic light scattering, and comparing the calculated values. When the calculated particle diameter distribution is not changed, it can be determined that the barotolerance and heat resistance are retained.

The particles as an active ingredient of the present disclosure have excellent cold resistance and drought resistance, and there is almost no change in particle diameter by drying and cooling to from -50 to -80°C. The cold resistance and drought resistance of particles can be determined as follows, for example. The particles are dispersed in ultrapure water, the dispersion liquid is freeze-dried (-50°C), and the obtained dry product is dispersed in ultrapure water again. The particle diameter distribution of the particles before and after the freeze-drying are calculated by dynamic light scattering, respectively, and comparing the calculated values. When the calculated particle diameter distribution is not changed, it can be determined that the cold resistance and drought resistance are retained. Furthermore, even after the lyophilizate is preserved at - 80°C for 7 days and dispersed in ultrapure water, and the diameter distribution of the particles is compared in the same manner, there is no change.

The particles as an active ingredient of the present disclosure can be produced by, for example, a method including a step of dissolving cell walls, and a step of separating particles from a lysate obtained by the above step.

Examples of the method for dissolving cell walls include heat treatment, supersonic treatment, treatment with a catabolic enzyme, and alkaline degradation treatment. These may be used alone or in combination. Among these, in view of efficiency and consideration of health, heat treatment and treatment with a catabolic enzyme are preferred, and heat treatment is particularly preferred.

The heat treatment includes, for example, immersing an organism having a cell wall in a liquid, heating the organism together with the liquid to dissolve components constituting the cell wall in the liquid, and thereby obtaining a lysate. More specifically, at first, an organism having a cell wall is provided. The organism may be in any state, but is preferably dried and crushed in view of efficiency. The provided organism is immersed in a liquid separately prepared, and usually heated at equal to or more than 60°C for equal to or more than 3 minutes, thereby obtaining a lysate. The longer the heating time is, the yield tends to increase. As the liquid, a liquid used as a solvent such as water or alcohol may be used alone, or as a mixture of two or more. In view of administrating the particles subcutaneously, nasally, orally, or the like, water or an aqueous liquid is preferably used in consideration of health.

Note that the term "dissolve components constituting the cell wall" means to disintegrate the cell wall structure by, for example, separating the basic structure and substrate, thereby forming a system in which said components are dispersed in liquid. The term also includes decomposing polysaccharides, which are one of said components, into a small size, and dispersing them in liquid.

Examples of the step of separating the particles as an active ingredient of the present disclosure from the lysate obtained by the above step include centrifugation, filter filtration, ultrafiltration, and ultracentrifugation. Among these, a suitable method is used depending on the species of the organism having a cell wall as a raw material. In particular, in view of the ease of operation, centrifugation and filter filtration are preferred, and in view of improving the purification degree, it is preferred to use these methods in combination.

Examples of the centrifugation includes: a method including centrifugation of the lysate at 10,000 to 1,000,000×g (depending on the particle size), thereby collecting the supernatant (crude separation step). Furthermore, for more increasing the purification degree, for example, the supernatant may be filtered through a filter having a pore size of 0.22 to 0.45 µm to obtain filtrate to obtain the filtrate (precise separation step).

As described above, the particles as an active ingredient of the present disclosure are excellent also in safety because a method for substituting terminal molecules of the component constituting a cell wall such as cellulose (e.g., a method using caustic soda, hydrochloric acid, or the like) is not used.

The immunoactivator of the present disclosure contains the thus obtained particles derived from a natural product as an active ingredient, and may comprise the particles alone, or may include other components.

Examples of the other components to be used with the particles as an active ingredient of the present disclosure include components having a pharmacological action, additives, and the like. Examples of the above additives include a base, carrier, solvent, dispersant, emulsifying agent, buffer, stabilizer, excipient, binder, disintegrant, lubricant, thickener, moisturizing agent, colorant, perfume, and chelating agent.

The immunoactivator of the present disclosure can induce maturation of dendritic cells, and thus can more efficiently enhance both humoral immunity and cellular immunity. Antigen-specific IgG production can be induced, and further CD8⁺T cell response, and antitumor immunity can also be induced. Thus, the immunoactivator of the present disclosure can more effectively activate immune function by synergistic effect of humoral immunity and cellular immunity.

The administration route of the immunoactivator of the present disclosure is not particularly limited as long as the desired effect can be obtained, and may be either enteral administration or parenteral administration.

Examples of the above enteral administration include nasal administration, oral administration, tube feeding, and enema administration. Examples of the above parenteral administration include transvenous administration, transarterial administration, pulmonary administration, intravascular administration, intramuscular administration, intracardiac administration, subcutaneous administration, intradermal administration, and intraperitoneal administration.

Among these, when using the subcutaneous administration or nasal administration as the administration route, immune response can be more reliably induced.

The immunoactivator of the present disclosure may be used in any form, either oral formulation or parenteral formulation.

Examples of the above oral formulation include tablets (including orally disintegrating tablets, chewable tablets, foaming tablets, troches, and jelly drops), pills, granules, fine granules, powders, hard capsules, soft capsules, dry syrups, liquid medicines (including drinkable preparation, suspending agents, and syrups), and jelly preparations.

Examples of the above parenteral formulation include injectable preparations (such as infusion injections, intravenous injections, intramuscular injections, subcutaneous injections, and intradermal injections), topical agents (such as ointments, gel patches, and lotions), suppositories, inhalations, eye drops, eye ointments, nasal preparations, eardrops, and liposome preparations.

The immunoactivator of the present disclosure can also be used as, for example, a component of food additives, food compositions (including health food, health builders, and nutrition supplement food (such as supplements)), and compositions such as cosmetics, in addition to medicines and reagents.

When the immunoactivator of the present disclosure is used as a component of food additives, health builders, nutrition supplement food (such as supplements), and the like, the form of the above food additives may be, for example, tablets (including orally disintegrating tablets, chewable tablets, foaming tablets, troches, and jelly drops), pills, granules, fine granules, powders, hard capsules, soft capsules, dry syrups, liquid medicines (including suspending agents and syrups), and jelly preparations.

When the immunoactivator of the present disclosure is used as a food composition, the form of the above food composition may be, for example, liquid, gel, or solid food, beverages (e.g., juice, cold beverages, tea, soup, and soy milk), salad oils, dressings, yoghurt, jellies, puddings, Furikake (seasoned dried food for sprinkling on rice), powdered milk for babies, cake mixes, dairy products (e.g., in the form of powder, liquid, gel, or solid), bread, and confectioneries (e.g., cookies).

### Vaccine adjuvant

The vaccine adjuvant of the present disclosure contains, as an active ingredient, particles that comprise cell-wall-derived polysaccharides and have a maximum diameter within a range of 1 to 800 nm. The above particles are the same as those described in the immunoactivator of the present disclosure, and have the similar effect.

The above vaccine adjuvant generally means a substance that is combinedly used with an antigen to enhance the antigenicity, for facilitating induction of immune response.

That is, vaccine, which is prepared by purifying a part of antigen components to be inoculated, generally has a weak effect. Therefore, use of an adjuvant is needed.

Moreover, vaccine development in recent years is not only directed to infectious diseases, but also to non-communicable diseases such as cancer, Alzheimer's disease, lifestyle diseases such as diabetes and hypertension, pollen or food allergy, and autoimmune diseases. However, the target of vaccine for the above non-communicable diseases usually cannot induce immunoreaction. Thus, it is difficult to obtain a desired effect.

Accordingly, development of an adjuvant that can induce a strong immunoreaction has been demanded even when the vaccine is targeted for the above non-communicable diseases.

The vaccine adjuvant of the present disclosure meets the above demand, and not only can be used in vaccine that is prepared by purifying a part of antigen components to be inoculated, but also can induce strong immunoreaction even when the vaccine is targeted for the non-communicable diseases as described above, because the induction of humoral immunity and/or cellular immunity can be enhanced.

The vaccine adjuvant of the present disclosure may be administered by any method as long as the induction of immune response is facilitated, and is preferably administered subcutaneously or nasally. From the viewpoint that systemic immunity and mucosal immunity can be induced with minimal invasion, nasal administration is preferred.

### Method for inducing immunity

The method for inducing immunity of the present disclosure includes a step of administrating the above immunoactivator. The step of administrating the above immunoactivator includes a step of administering the above immunoactivator, and thereafter, additionally administering the above immunoactivator after the lapse of a certain period.

In the method for inducing immunity of the present disclosure, as long as the immune response can be induced, the above immunoactivator may be administered by any method in the step of administering the above immunoactivator. In particular, from the viewpoint that administration can be easily performed, the above immunoactivator is preferably administered subcutaneously or nasally. From the viewpoint that systemic immunity and mucosal immunity can be induced with minimal invasion, nasal administration is preferred.

### EXAMPLES

Hereinafter, the present disclosure will be described more specifically with reference to Examples. However, the present disclosure is not limited to the following Examples within the scope of the present disclosure.

Note that "part" and "%" in examples are based on weight.

At first, as organisms having a cell wall, licorice (crude drug pieces for decoction: Licorice P of Tochimoto, product of Tochimoto Tenkaido Co., Ltd.), yeast (dry product: natural beer yeast, product of Nippon Garlic Corporation), and lactic acid bacteria (powder: lactic acid bacteria powder KA-18, product of Biolabo Co., Ltd.) were prepared. Then, particles as an active ingredient of the present disclosure were produced as follows.

### Particles derived from licorice

One hundred (100) grams of chopped licorice (dry product) was put in 500 mL of ultrapure water, and the mixture was boiled for 50 minutes to prepare a licorice decoction. After allowing to cool, the decoction was centrifuged at 20000×g for 60 minutes for the purpose of removing coarse fractions. The supernatant was collected, and the collected supernatant was mixed with an equivalent amount of ethanol in volume ratio for the purpose of purifying nanoparticles. After the mixture was centrifuged at 20000×g for 20 minutes, the supernatant was removed. The step of mixing the supernatant after the above centrifugation for 60 minutes with an equivalent amount of ethanol in volume ratio and the subsequent processes were repeatedly performed twice. Thereafter, pellets remained on the bottom surface of the container were dispersed again in ultrapure water, and dialysis was performed for the purpose of removing the ethanol mixed in the above step in the ultrapure water by using a dialysis membrane with 50 kDa of molecular weight cut off (MWCO) for 1 day. The resultant was lyophilized to obtain particles.

For the obtained particles, FIG. 2 shows the particle size distribution, and Table 1 below shows the recovery amount, average particle diameter, polydispersion index, and zeta potential.

**Table 1**

| Particles derived from licorice | | | | |
|---|---|---|---|---|
| | Recovery amount (mg) | Average particle diameter (nm) | Polydispersion index | Zeta potential (mV) |
| Particles derived from licorice | 219 | 159 | 0.374 | -22.0 |

### Particles derived from yeast

One hundred (100) grams of yeast (dry product) was put in 1 L of ultrapure water, and the mixture was heated at 90°C for 3 hours. After allowing to cool, the mixture was centrifuged at 12300×g for 30 minutes for the purpose of removing coarse fractions. The supernatant was collected, and the mixture was centrifuged again at 140000×g for 60 minutes. The supernatant was removed, and pellets, which were aggregates of particles remained on the bottom surface of the container, were dispersed again in ultrapure water, and the resultant was lyophilized to obtain particles.

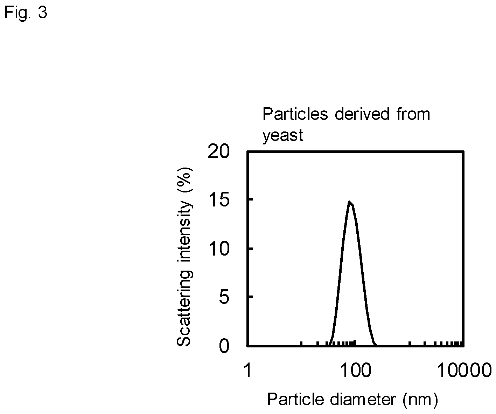

For the obtained particles, FIG. 3 shows the particle size distribution, and Table 2 below shows the recovery amount, average particle diameter, and polydispersion index.

**Table 2**

| Particles derived from yeast | | | |
|---|---|---|---|
| | Recovery amount (mg) | Average particle diameter (nm) | Polydispersion index |
| Particles derived from yeast | 786.6 | 82.1 | 0.151 |

### Particles derived from lactic acid bacteria

Ten (10) grams of lactic acid bacteria (powder) was put in 100 mL of ultrapure water, and the mixture was heated at 90°C for 3 hours. After allowing to cool, the mixture was centrifuged at 12300×g for 30 minutes for the purpose of removing coarse fractions. The supernatant was collected, and the mixture was centrifuged again at 140000×g for 60 minutes. The supernatant was removed, and pellets, which were aggregates of particles remained on the bottom surface of the container, were dispersed again in ultrapure water, and the resultant was lyophilized to obtain particles.

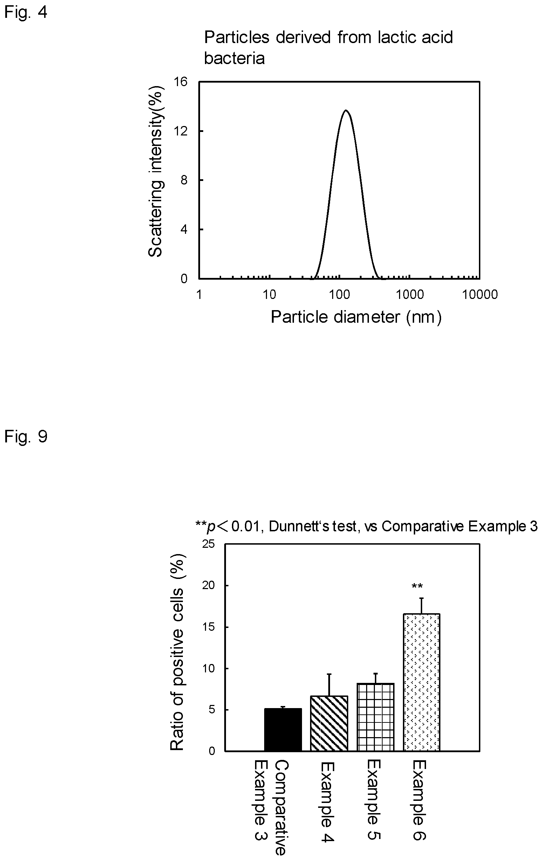

For the obtained particles, FIG. 4 shows the particle size distribution, and Table 3 below shows the recovery amount, average particle diameter, and polydispersion index.

**Table 3**

| Particles derived from lactic acid bacteria | | | |
|---|---|---|---|
| | Recovery amount (mg) | Average particle diameter (nm) | Polydispersion index |
| Particles derived from lactic acid bacteria | 78.07 | 112.9 | 0.155 |

For the above obtained particles, at first, a cell experiment using a mouse dendritic cell line (DC2.4 cells) was performed, and maturation of the dendritic cells was examined. Then, an animal experiment (subcutaneous immunization) was performed on the above obtained particles to examine each item of antigen-specific antibody production, cell response, and induction of antitumor immunity (1) and (2). Each experiment was performed under the following conditions.

### 1. Cell experiment: Maturation of dendritic cells

### (1-1) Particles derived from licorice

### Example 1, Comparative Example 1, and Reference Example 1

In Example 1, the above obtained particles derived from licorice were reacted with the mouse dendritic cell line (DC2.4 cells, 5×10⁵ cells/2 mL) such that the particles were 100 µg/mL. After culturing the resultant at 37°C for 24 hours, the levels of expression for the dendritic cell maturation markers CD40, CD80, and CD86 were measured.

On the other hand, in Comparative Example 1A, a non-treated mouse dendritic cell line was used. In Comparative Example 1B, the mouse dendritic cell line with which 100 µg/mL of the licorice decoction (before purification) obtained in the above step for producing the particles derived from licorice was reacted was used. As a positive control, the mouse dendritic cell line with which 0.1 µg/mL of lipopolysaccharide (LPS) was reacted was used in Reference Example 1. The level of expression of each maturation marker was measured in the same manner as in Example 1.

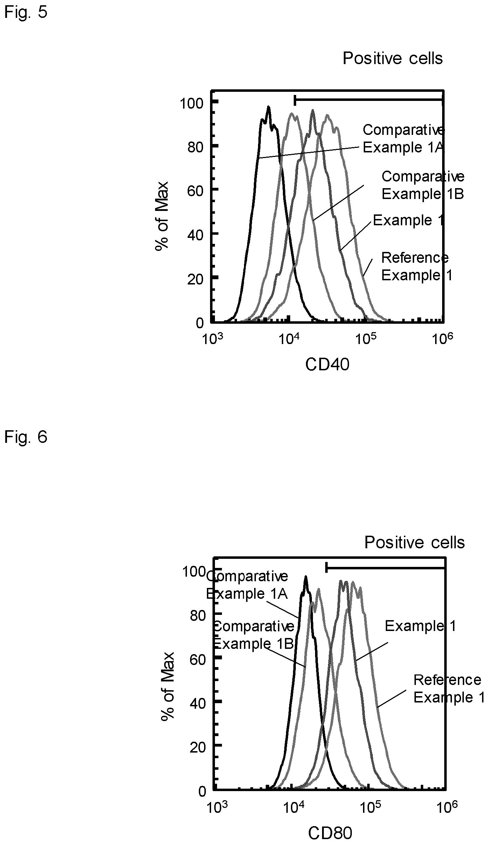

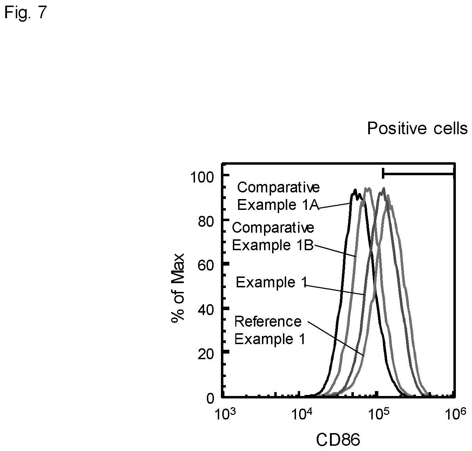

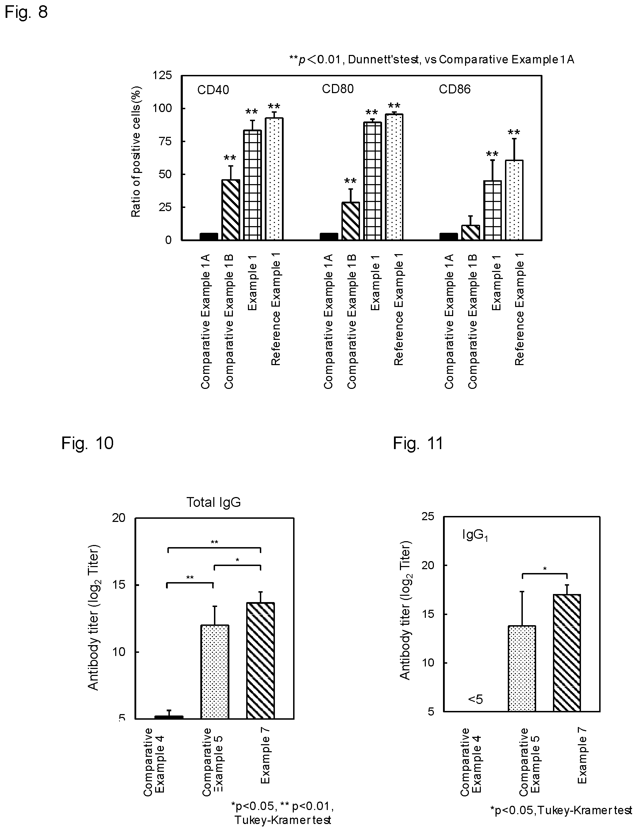

These results were analyzed with flow cytometry. FIG. 5 shows the results of CD40, FIG. 6 shows the results of CD80, and FIG. 7 shows the results of CD86. FIG. 8 shows the comparison of these results.

### (1-2) Particles derived from yeast

### Example 2,3, Comparative Example 2, and Reference Example 2

In Examples 2 and 3, the above obtained particles derived from yeast were reacted with the mouse dendritic cell line (DC2.4 cells, 5×10⁵ cells/2 mL) at the concentration shown in Table 4 below, respectively. After culturing the resultant at 37°C for 24 hours, the levels of expression of the dendritic cell maturation markers CD40, CD80, and CD86 were measured.

On the other hand, in Comparative Example 2, a non-treated mouse dendritic cell line was used. As a positive control, the mouse dendritic cell line with which 0.1 µg/mL of lipopolysaccharide (LPS) was reacted was used in Reference Example 2. The level of expression of each maturation marker was measured in the same manner as in Examples 2 and 3.

These results were analyzed with flow cytometry. Table 4 below collectively shows the expression ratio of each maturation marker.

**Table 4**

| Expression ratio of each maturation marker | | | | | |
|---|---|---|---|---|---|
| After the lapse of 24 hours at 37°C | | | CD40(%) | CD80(%) | CD86(%) |
| Comparative Example 2 | None | | 4.76 | 4.94 | 5.18 |
| Example 2 | Yeast particles | 10 µg/mL | 23.0 | 13.4 | 10.9 |
| Example 3 | Yeast particles | 100 µg/mL | 51.0 | 39.0 | 18.2 |
| Reference Example 2 | LPS | 0.1 µg/mL | 66.8 | 38.2 | 13.7 |

### (1-3) Particles derived from lactic acid bacteria

### Examples 4 to 6, and Comparative Example 3

In Examples 4 to 6, the above obtained particles derived from lactic acid bacteria were reacted with the mouse dendritic cell line (DC2.4 cells, 5×10⁵ cells/2 mL) such that the particles were 1, 10, or 100 µg/mL, respectively. After culturing the resultant at 37°C for 24 hours, the level of expression of the dendritic cell maturation marker CD40 was measured.

On the other hand, in Comparative Example 3, a non-treated mouse dendritic cell line was used, and the level of expression of each maturation marker was measured in the same manner as in Examples 4 to 6.

These results were analyzed with flow cytometry. FIG. 9 shows the results of CD40.

As shown in FIGs. 5 to 9 and Table 4 above, any of Examples show equivalent levels of expression as LPS, the positive control. Thus, it is recognized that the immunoactivator of the present disclosure affects maturation of dendritic cells, which is important at an initial stage of activation of acquired immunity such as antibody production.

### 2. Animal experiment (subcutaneous immunization): Antigen-specific antibody production

### (2-1) Particles derived from licorice

### Example 7, and Comparative Examples 4 and 5

In Example 7, mice (C57BL/6J, 6 weeks old, female) were immunized subcutaneously at the back with 100 µg of the above obtained particles derived from licorice and 100 µg of hen egg white ovalbumin (OVA) as a model antigen. The above subcutaneous immunization was performed again 7 days later (number of subcutaneous immunizations: 2 in total). Seven days after the day of final subcutaneous immunization, blood was collected, and values of total IgG, IgG₁, and IgG_{2c} (antibody titers) were measured.

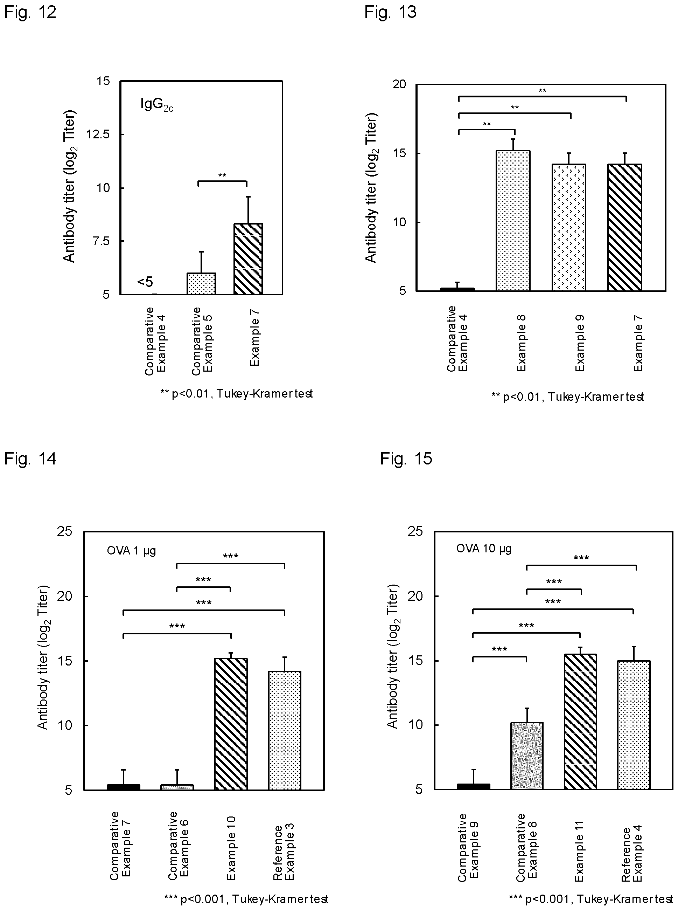

On the other hand, unimmunized mice were used in Comparative Example 4. In Comparative Example 5, mice were used, which were immunized in the same manner as in Example 7 except that the particles derived from licorice were not used. Then, the antibody titers were measured in the same manner as in Example 7. FIG. 10 shows the results of the total IgG, FIG. 11 shows the results of IgG₁, and FIG. 12 shows the results of IgG_{2C}.

As shown in FIG. 10, antigen-specific IgG could be induced by immunization using the antigen and the particles derived from licorice in combination. Further, from the results shown in FIGs. 11 and 12, by subclass analysis of the induced total IgG, induction of antigen-specific IgG₁ and IgG_{2C} were confirmed. On the other hand, when immunization was performed only with the antigen, a Th2 type immune response was superior (see Comparative Example 5). As described above, it was revealed that Example 7 is useful as an immunoactivator capable of efficiently inducing a Th1 type immune response.

By the way, antigen information presented by antigen-presenting cells, which are mainly dendritic cells, is transmitted to T cells, thereby inducing antigen-specific acquired immunity. The above acquired immunity is divided into humoral immunity and cellular immunity. Humoral immunity (Th2 type) is to produce antibodies against action-targets (such as pathogens and cancer cells), and cellular immunity is to eliminate action-targets based on direct damage to the action-targets by cytotoxic T-lymphocytes. Therefore, it is desirable that the immunoactivator can induce both humoral immunity and cellular immunity. Among antibodies, IgG_{2c} is an antibody produced in response to cellular immunity (Th1) type cytokines.

### (2-2) Particles derived from licorice

### Examples 8 and 9

In Example 8, mice were used, which were immunized in the same manner as in Example 7 except that the amount of OVA as an antigen was changed to 1 µg. In Example 9, mice were used, which were immunized in the same manner as in Example 7 except that the amount of OVA was changed to 10 µg. Then, the total IgG were measured in the same manner as in Example 7. The results are shown in FIG. 13 with the total IgG in Example 7 and Comparative Example 4.

FIG. 13 shows that, even when the amount of the antigen was 1 µg, antibody production equivalent to that obtained with 100 µg of the antigen was induced. The smaller the amount of antigen derived from pathogen or cancer cells administered at the time of immunization, the more preferable, in view of the time required for preparation, economical cost, safety, and the like. It is found that the immunoactivator of the present disclosure is excellent in terms of the time consumed, economical cost, and safety because: when the amount of antigen is reduced to one hundredth, i.e., from 100 µg to 1 µg, the antibody titer equivalent to that obtained with 100 µg of antigen can be obtained; and high immune function is observed.

### (2-3) Particles derived from yeast

Examples 10 and 11, Comparative Examples 6 to 9, and Reference Examples 3 and 4

In Examples 10 and 11, mice (C57BL/6J, 6 weeks old, female) were immunized subcutaneously at the back with 100 µg of the above obtained particles derived from yeast and OVA (1 µg or 10 µg). The above subcutaneous immunization was performed again 7 days later (number of subcutaneous immunizations: 2 in total). Seven days after the day of final subcutaneous immunization, blood was collected, and the total IgG antibody titer was measured.

In Comparative Example 6, mice were used, which were immunized in the same manner as in Example 10 above (OVA 1µg) except that the particles derived from yeast were not used. In Comparative Example 7, unimmunized mice were used. In Reference Example 3, as a positive control, 1 mg of a formulation of aluminum hydroxide and magnesium hydroxide (product of Thermo Fisher Scientific, Inc., Imject Alum Adjuvant) (hereinafter, referred to as "Alum") was used in place of the particles derived from yeast.

Furthermore, in Comparative Example 8, mice were used, which were immunized in the same manner as in Example 11 above (OVA 10 µg) except that the particles derived from yeast were not used. In Comparative Example 9, unimmunized mice were used. In Reference Example 4, as a positive control, Alum (1mg) was used in place of the particles derived from yeast.

For these mice, the antibody titers were measured in the same manner as in Examples 10 and 11, respectively, and the total IgG antibody titers are collectively shown in FIGs. 14 and 15. Note that individuals having an antibody titer of equal to or less than 5 are calculated as having an antibody titer of 5.

As shown in Figs. 14 and 15, when mice were immunized with the particles derived from yeast, antibody titers were significantly increased in both Examples than Comparative Examples even when the amount of the antigen was either 1 µg or 10 µg. These antibody titers are equivalent to those obtained in the mice immunized with Alum, the positive control. Both Examples show high antibody production enhancing effect.

### 3. Animal experiment (subcutaneous immunization): T cell response

### (3-1) Particles derived from licorice

### Examples 12, and Comparative Examples 10 and 11

In Example 12, mice (C57BL/6J, 6 weeks old, female) were immunized subcutaneously at the back with 100 µg of the above obtained particles derived from licorice and 100 µg of hen egg white ovalbumin (OVA) as a model antigen. The above subcutaneous immunization was performed again 7 days later (number of subcutaneous immunizations: 2 in total). Seven days after the day of final subcutaneous immunization, splenocytes were collected.

On the other hand, in Comparative Example 10, unimmunized mice were used. In Comparative Example 11, mice were used, which were immunized in the same manner as in Example 12 above except that the licorice particles were not used. Then, splenocytes were collected in the same manner as in Example 12.

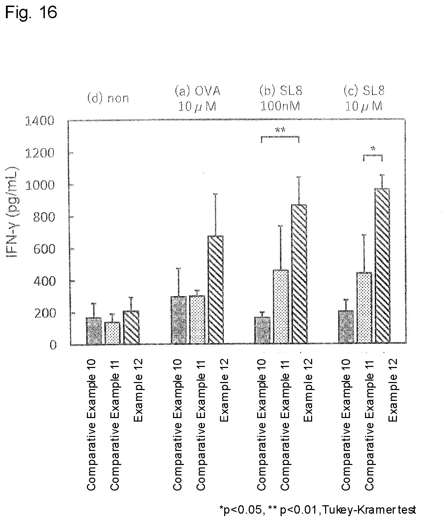

For these Examples and Comparative Examples, stimulation was performed on the splenocytes under the conditions shown in (a) to (d) below, respectively. After 3 days, the amount of IFN-γ in the cell supernatant was measured with ELISA method. The results are collectively shown in FIG. 16.
(a) OVA 10µM
(b) OVA-derived MHC class I epitope peptide (SL8) 100 nM
(c) OVA-derived MHC class I epitope peptide (SL8) 10 µM
(d) No stimulation (non)

As shown in FIG. 16, Example 12 showed higher IFN-γ production with any restimulation than Comparative Examples 10 and 11, and in particular, it was found that antigen-specific CD8⁺T cell response can be induced.

T cells having an antigen-specific T-cell receptor are activated by restimulation of antigen, thereby producing IFN-γ. In particular, stimulation with OVA-derived MHC class I epitope peptide (SL8) can specifically activate CD8⁺T cells. Thus, the immunoactivator of the present disclosure can also exhibit excellent effect on cellular immunity.

### (3-2) Particles derived from yeast

Examples 13 and 14, Comparative Examples 12 to 15, and Reference Examples 5 and 6

In Examples 13 and 14, mice (C57BL/6J, 6 weeks old, female) were immunized subcutaneously at the back with 100 µg of the particles derived from yeast obtained in the above step and 1 µg of OVA (Example 13) or 10 µg of OVA (Example 14). The above subcutaneous immunization was performed again 7 days later (number of subcutaneous immunizations: 2 in total). Seven days after the day of final subcutaneous immunization, splenocytes were collected.

On the other hand, in Comparative Example 12, unimmunized mice were used. In Comparative Example 13, mice were used, which were immunized in the same manner as in Example 13 above except that the particles derived from yeast were not used. In Reference Example 5, as a positive control, Alum (1mg) was used in place of the particles derived from yeast.

In Comparative Example 14, unimmunized mice were used. In Comparative Example 15, mice were used, which were immunized in the same manner as in Example 14 above except that the particles derived from yeast were not used. In Reference Example 6, as a positive control, Alum (1mg) was used in place of the particles derived from yeast.

From these mice, splenocytes were collected in the same manner as in Examples 13 and 14, respectively.

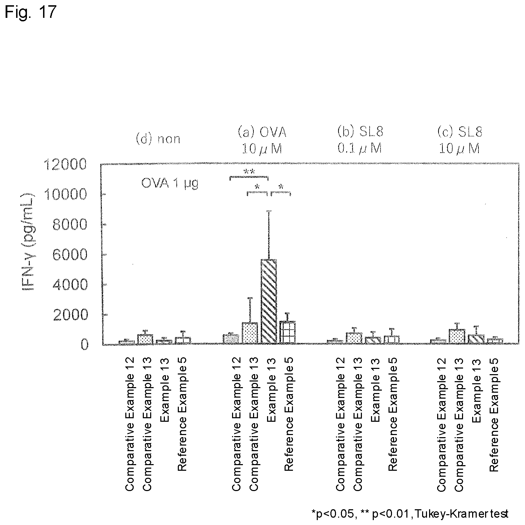

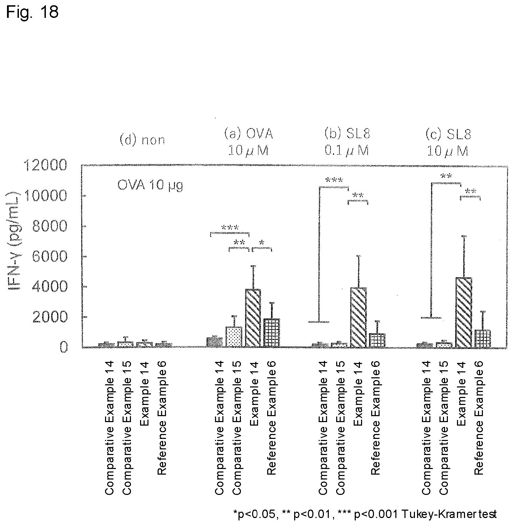

For these Examples, Comparative Examples, and Reference Examples, stimulation was performed on the splenocytes under the conditions shown in (a) to (d) below. Then, after 3 days, the amount of IFN-γ in the cell supernatant was measured with ELISA method. The results are collectively shown in FIGs. 17 and 18.
(a) OVA 10µM
(b) OVA-derived MHC class I epitope peptide (SL8) 0.1 µM
(c) OVA-derived MHC class I epitope peptide (SL8) 10 µM
(d) No stimulation (non)

As shown in FIG. 17, Example 13 showed higher T cell response than Comparative Examples 12 and 13 by restimulation with 10 µM of OVA after immunization with 1 µg of OVA. Moreover, as shown in FIG. 18, Example 14 showed high CD8⁺T cell response by either SL8 restimulation after immunization with OVA (10µg). The T cell response was not observed in Reference Examples 5 and 6, and thus is considered one of useful effects of the immunoactivator of the present disclosure.

### 4. Animal experiment (subcutaneous immunization): Induction of antitumor immunity (1)

### Example 15

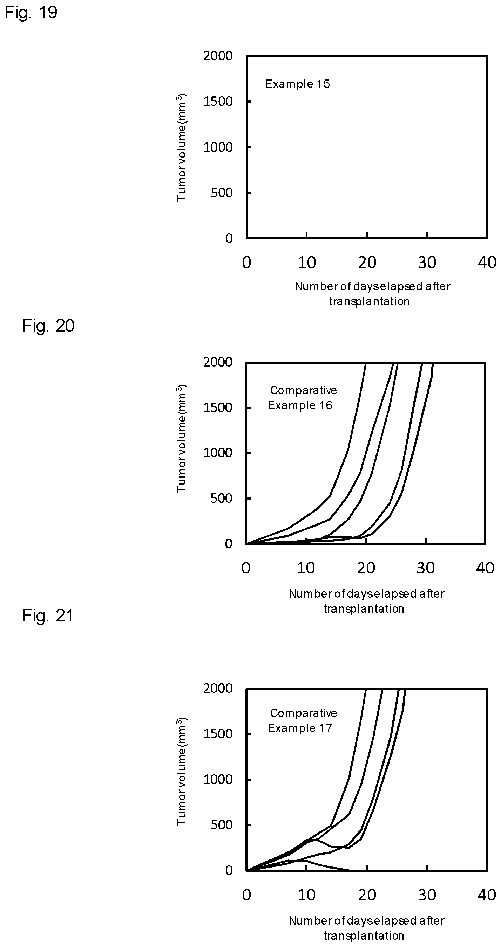

In Example 15, mice (C57BL/6J, 6 weeks old, female) were immunized subcutaneously at the back with 100 µg of the particles derived from licorice obtained in the above step and 100 µg of OVA. The above subcutaneous immunization was performed again 7 days later (number of subcutaneous immunizations: 2 in total). Seven days after the day of final subcutaneous immunization, 1×10⁶ cells of an OVA-expressed lymphoma cell line (E.G7-OVA) were transplanted to the back of the mice. Then, change in the tumor volume (mm³) with the number of days elapsed after the transplantation was measured. The measurement results are shown in FIG. 19.

### Comparative Examples 16 and 17

In Comparative Example 16, mice were used, which were immunized in the same manner as in Example 15 except that the particles derived from licorice were not used. In Comparative Example 17, unimmunized mice were used. Thus, changes in the tumor volume were measured in the same manner as in Example 15. FIG. 20 shows the results of Comparative Example 16, and FIG. 21 shows the results of Comparative Example 17.

As shown in FIG. 19, Example 15 did not show tumor engraftment or tumor growth in all mice (rejection rate 5/5). Thus, it can be said that efficacy of the immunoactivator of the present disclosure as an adjuvant in immunotherapy was confirmed. Meanwhile, Comparative Examples 16 and 17 showed tumor volume increases as shown in FIG. 20 (rejection rate 0/5) and FIG. 21 (rejection rate 1/5).

### 5. Animal experiment (subcutaneous immunization): Induction of antitumor immunity (2)

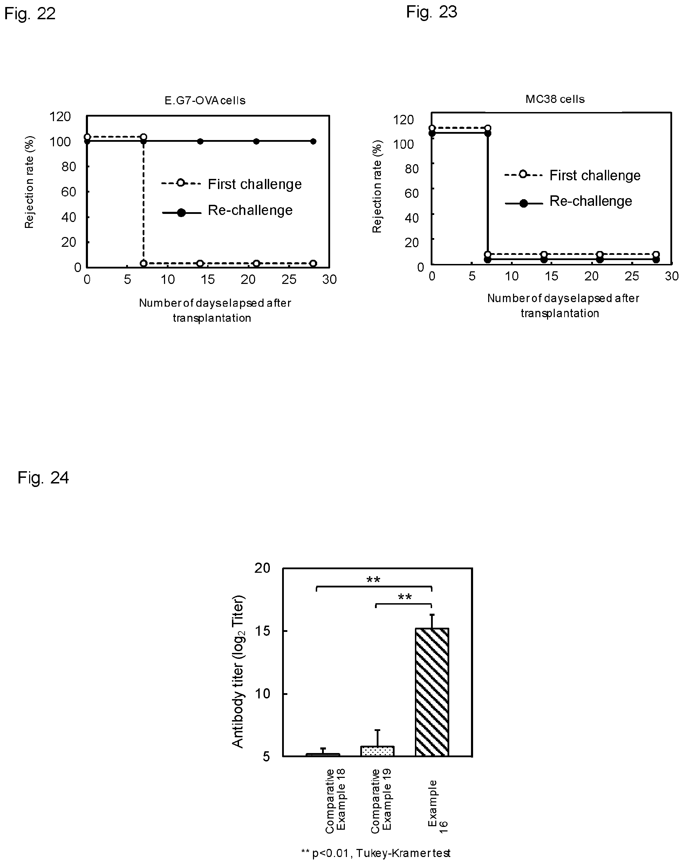

(5-1)
To the mice of Example 15 in which the OVA-expressed lymphoma cell line (E.G7-OVA) was not engrafted, 1×10⁶ cells of the OVA-expressed lymphoma cell line (E.G7-OVA) were transplanted again to the back when 70 days have passed after the transplantation of the above OVA-expressed lymphoma cell line (E.G7-OVA). Thus, formation of immunological memory was evaluated. The measurement results are shown in FIG. 22.

As shown in FIG. 22, the mice that rejected the cancer cells in Example 15 rejected cancer cells again in retransplantation of the cancer cells after 70 days. Thus, it can be said that the immunoactivator of the present disclosure can induce immunological memory against cancer cells.

(5-2)
To the mice of Example 15 in which the OVA-expressed lymphoma cell line (E.G7-OVA) was not engrafted, 1×10⁶ cells of a colon cancer cell line (MC38) were transplanted to the back when 70 days have passed after the transplantation of the above OVA-expressed lymphoma cell line (E.G7-OVA). Thus, formation of cell-specific immunological memory was evaluated. The measurement results are shown in FIG. 23.

As shown in FIG. 23, the mice that rejected the OVA-expressed lymphoma cell line (E.G7-OVA) transplanted in Example 15 did not reject the colon cancer cell line (MC38).

On the other hand, as shown in FIG. 22, the mice that rejected the OVA-expressed lymphoma cell line (E.G7-OVA) transplanted in Example 15 rejected the OVA-expressed lymphoma cell line (E.G7-OVA) again. Thus, it can be said that the immune response enhancing effect obtained by the immunoactivator of the present disclosure is antigen-specific and cell-specific, and does not induce immune response other than in the action target.

As described above, the above particles were demonstrated to activate immune function.

Accordingly, the immunoactivator containing the above particles as an active ingredient is derived from cell-walls (derived from a natural product), capable of enhancing induction of both humoral immunity and cellular immunity, and thus has both efficacy of activating immune function and safety for human bodies.

Furthermore, to the above obtained particles, an animal experiment (nasal immunization) was performed to evaluate each item of antigen-specific antibody production, and induction of antitumor immunity. Each experiment was performed under the following conditions.

### 6. Animal experiment (nasal immunization): Antigen-specific antibody production

### Example 16

In Example 16, mice (C57BL/6J, 6 weeks old, female) were nasally immunized with the above obtained particles derived from licorice (100 µg) and OVA (100 µg). The above nasal immunization was performed again 7 days later (number of nasal immunizations: 2 in total). Seven days after the day of final nasal immunization, blood was collected, and the total IgG antibody titer was measured.

### Comparative Examples 18 and 19

In Comparative Example 18, unimmunized mice were used. In Comparative Example 19, mice were used, which were immunized in the same manner as in Example 16 above except that the particles derived from licorice were not used. The total IgG antibody titers measured in the same manner as in Example 16 are shown in FIG. 24.

As shown in FIG. 24, Example 16 showed significantly increased antibody titer as compared with Comparative Examples 18 and 19. Thus, the immunoactivator of the present disclosure is recognized to have high antibody production enhancing effect also in nasal immunization.

### 7. Animal experiment (nasal immunization): Induction of antitumor immunity (1)

### Example 17

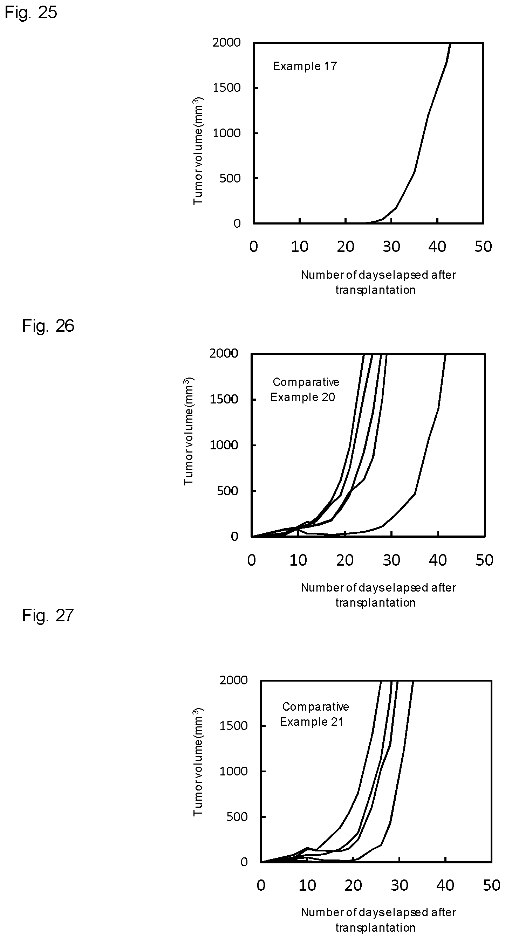

Mice (C57BL/6J, 6 weeks old, female) were nasally immunized with the above particles derived from licorice (100 µg) and OVA (100 µg). The above nasal immunization was performed again 7 days later (number of nasal immunizations: 2 in total). Seven days after the day of final nasal immunization, 1×10⁶cells of the OVA-expressed lymphoma cell line (E.G7-OVA) were transplanted to the back of the mice. Thus, change in the tumor volume (mm³) with the number of days elapsed after the transplantation was measured. FIG. 25 shows the measurement results.

### Comparative Examples 20 and 21

In Comparative Example 20, mice were used, which were immunized in the same manner as in Example 17 except that the licorice particles were not used. In Comparative Example 21, unimmunized mice were used. Thus, changes in the tumor volume (mm³) were measured in the same manner as in Example 17. FIG. 26 shows the results of Comparative Example 20, and FIG. 27 shows the results of Comparative Example 21.

As shown in FIG. 25, in Example 17, most mice rejected the cancer cells (rejection rate 4/5), while Comparative Examples 20 and 21 showed tumor volume increase (see FIG. 26 (rejection rate 0/5) and FIG. 27 (rejection rate 1/5). Thus, the immunoactivator of the present disclosure is recognized to have efficacy as an adjuvant in cancer immunotherapy also in nasal immunization.

### 8. Animal experiment (nasal immunization): Induction of antitumor immunity (2)

(8-1)
To the mice of Example 17 in which the OVA-expressed lymphoma cell line (E.G7-OVA) was not engrafted, 1×10⁶ cells of the OVA-expressed lymphoma cell line (E.G7-OVA) were transplanted again to the back when 70 days have passed after the transplantation of the above OVA-expressed lymphoma cell line (E.G7-OVA). Then, the tumor volume was measured over time (Re-challenge).

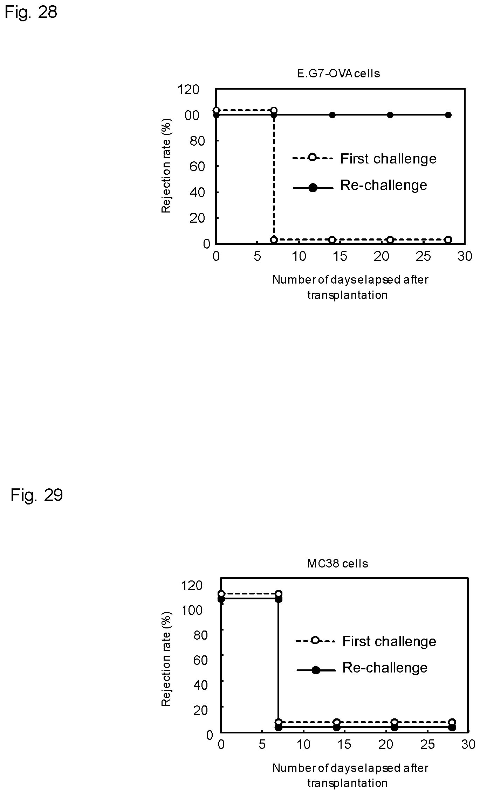

As controls, 1×10⁶cells of the OVA-expressed lymphoma cell line (E.G7-OVA) were transplanted to the back of unimmunized mice, and the tumor volume was measured over time (First-challenge). From these results, rejection rate against transplantation of the OVA-expressed lymphoma cell line (E.G7-OVA) was calculated. The results are shown in FIG. 28.

(8-2)
To the mice of Example 17 in which the OVA-expressed lymphoma cell line (E.G7-OVA) was not engrafted, 1×10⁶ cells of the colon cancer cell line (MC38) were transplanted to the back when 70 days have passed after the transplantation of the above OVA-expressed lymphoma cell line (E.G7-OVA). Then, the tumor volume was measured over time (Re-challenge).

As controls, 1×10⁶cells of the colon cancer cell line (MC38) were transplanted to the back of unimmunized mice, and the tumor volume was measured over time (First-challenge). From these results, rejection rate against transplantation of the OVA-expressed lymphoma cell line (E.G7-OVA) was calculated. The results are shown in FIG. 29.

As shown in FIG. 28, the mice that rejected the cancer cells in Example 17 rejected cancer cells again, in which the cancer cells were retransplanted when 70 days have passed after the transplantation. Thus, the immunoactivator of the present disclosure shows induction of immunological memory against cancer cells also in nasal immunization.

On the other hand, as shown in FIG. 29, the mice that rejected the cancer cells in Example 17 did not reject the colon cancer cell line (MC38) without OVA expression. Therefore, it can be said that the immune response enhancing effect obtained by nasal immunization of the immunoactivator of the present disclosure is antigen-specific and cell-specific, and does not induce immune response other than in the action target.

As described above, the above particles can activate immune function also in nasal immunization, and thus it was shown that the immunoactivator containing the above particles as an active ingredient is also effective in nasal immunization. Furthermore, when the effect is obtained through the nose, the effect may be obtained through the vagina, eye, oral cavity, and the like, which are the same transmucosal administration.

That is, the immunoactivator of the present disclosure can induce not only systemic immunity response but also mucosal immunity response, and thus can be expected to have an effect against pathogen such as those infected through mucosal surface. Further, the nasal immunization is noninvasive different from subcutaneous immunization, and medical waste can be reduced. Thus, the nasal immunization is also advantageous in view of practical use.

The specific embodiments of the present disclosure have been shown in the above Examples, but the above Examples are merely illustrative and should not be construed to be limiting. Various modifications obvious to a person skilled in the art are intended to be within the scope of the present disclosure.

### INDUSTRIAL APPLICABILITY

The immunoactivator of the present disclosure is industrially useful as an immunoactivator having both efficacy of activating immune function and safety for human bodies. The vaccine adjuvant of the present disclosure is industrially useful as a vaccine adjuvant to be used for cancer immunotherapy as well as various infectious diseases. Furthermore, the method for inducing immunity of the present disclosure is industrially useful because it is highly safe for human bodies and can be performed only via simple steps.

### REFERENCE SIGNS LIST

- A: Particles

## Claims

1. An immunoactivator comprising particles having a maximum diameter within a range of 1 to 800 nm as an active ingredient, wherein the particles comprise cell-wall-derived polysaccharides.

2. The immunoactivator according to claim 1, wherein the particles are derived from at least one selected from the group consisting of licorice, yeast, and lactic acid bacteria.

3. The immunoactivator according to claim 1 or 2, wherein the immunoactivator is capable of at least one of infection treatment and cancer immunotherapy.

4. The immunoactivator according to any one of claims 1 to 3, wherein the immunoactivator induces at least one of humoral immunity and cellular immunity.

5. The immunoactivator according to any one of claims 1 to 4, wherein the immunoactivator is capable of being administered by at least one administration method selected from the group consisting of subcutaneous administration, intradermal administration, intramuscular administration, pulmonary administration, intravascular administration, nasal administration, and oral administration.

6. A vaccine adjuvant comprising particles having a maximum diameter within a range of 1 to 800 nm as an active ingredient, wherein the particles comprise cell-wall-derived polysaccharides.

7. The vaccine adjuvant according to claim 6, wherein the vaccine adjuvant is capable of being administered by at least one administration method selected from the group consisting of subcutaneous administration, intradermal administration, intramuscular administration, pulmonary administration, intravascular administration, nasal administration, and oral administration.

8. A method for inducing immunity comprising administering the immunoactivator according to any one of claims 1 to 5 to a subject in need thereof.

9. The method for inducing immunity according to claim 8, wherein administering the immunoactivator comprises administration of the immunoactivator by at least one administration method selected from the group consisting of subcutaneous administration, intradermal administration, intramuscular administration, pulmonary administration, intravascular administration, nasal administration, and oral administration.
